# EUROPEAN PATENT SPECIFICATION

(11) **EP 3 113 768 B1**
(45) Date of publication and mention of the grant of the patent: **26.09.2018**
(21) Application number: 15715826.2
(22) Date of filing: 04.03.2015
(51) Int. Cl.: A61K 31/137, A61K 31/40, A61K 31/4174, A61K 31/4453, A61K 31/495, A61K 31/5375, A61K 31/54, G01N 33/574, A61P 25/30, A61P 25/02, A61P 29/00

(54) **USE OF ARYLALKANOLAMINES AS SIGMA-1 RECEPTOR ANTAGONISTS**
VERWENDING VON ARYLALKANOLAMINEN ALS SIGMA-1 REZEPTORANTAGONISTEN
UTILISATION D'ARYLALCANOLAMINES EN TANT QU'ANTAGONISTES DE RÉCEPTEUR SIGMA-1

(30) Priority: 05.03.2014 IT MI20140338
(43) Date of publication of application: 11.01.2017
(73) Proprietor: Universita' Degli Studi Di Pavia, 27100 Pavia (IT)
(72) Inventor: COLLINA, Simona, I-27100 Pavia (IT); ROSSI, Daniela, I-27100 Pavia (IT); MARRA, Annamaria, I-27100 Pavia (IT); PEVIANI, Marco, I-27100 Pavia (IT); CURTI, Daniela, I-27100 Pavia (IT)
(74) Representative: Schmauder & Partner AG Patent- & Markenanwälte VSP
(86) International application number: PCT/IB2015/051573
(87) International publication number: WO 2015/132733

(56) References cited:
- WO-A1-03/106443
- BE-A- 642 084
- GB-A- 1 434 826
- US-A- 4 916 156
- COLLINA S ET AL: "Sigma receptor modulators: A patent review", EXPERT OPINION ON THERAPEUTIC PATENTS, INFORMA HEALTHCARE, GB, vol. 23, no. 5, 1 May 2013 (2013-05-01), pages 597-613, XP009170155, ISSN: 1354-3776, DOI: 10.1517/13543776.2013.769522 cited in the application
- N. K. RAMAKRISHNAN ET AL: "Small-Animal PET with a -Ligand, 11C-SA4503, Detects Spontaneous Pituitary Tumors in Aged Rats", THE JOURNAL OF NUCLEAR MEDICINE, vol. 54, no. 8, 19 June 2013 (2013-06-19), pages 1377-1383, XP055147109, ISSN: 0161-5505, DOI: 10.2967/jnumed.112.115931 cited in the application

## Description

### FIELD OF THE INVENTION

The present invention relates to arylalkanolamine compounds of formula (I) as defined in the claims with sigma-1 receptor antagonist activity for use in the prevention or treatment of conditions selected from the abuse of psychotropic substances such as cocaine or amphetamines, and pain.

### PRIOR ART

Sigma (σ) receptors were identified at the end of the 1980s. In the 1990s, two receptor subtypes were identified, named sigma-1 (σ-1) and sigma-2 (σ-2), which show a diverse pharmacological and physiological profile.

The σ-1 receptor is a protein comprising 223 amino acids, located on the membrane of the mitochondrial endoplasmic reticulum. The σ-2 receptor has not yet been cloned.

Under resting conditions, the σ-1 receptor forms a complex with the BiP protein. Cellular stress conditions and/or interaction with σ-1 receptor ligands bring about dissociation of the complex between the σ-1 receptor and the BiP protein and translocation of the σ-1 receptor to other cell compartments, for example to the plasma membrane and the nuclear membrane. The σ-1 receptor is thus considered to be a chaperone protein, which acts as a modulator between the various organelles present inside the cell.

The σ-1 receptor is widely distributed in the central nervous system, in particular in the spinal cord, in the cerebellum, in the hippocampus, in the hypothalamus and in the cerebral cortex. For this reason, the role of the σ-1 receptors in central nervous system pathologies is now of great interest and is the subject of numerous studies.

The role of the sigma-1 receptor agonists and antagonists was reviewed by Maurice T. and Su T.-P. in the article "The pharmacology of sigma-1 receptors" (Pharmacol. Ther., 2009, Vol. 124(2), pages 195-206). σ-1 receptor ligands with agonist activity are of interest for treating depression, states of amnesia, Alzheimer's disease and neuronal degeneration of the retina. σ-1 receptor ligands with agonist activity are under study for treating cocaine and amphetamine abuse, neuropathic pain and certain types of cancer, in particular breast cancer.

Ramakrishnan N.K. et al. have described the use of a σ-1 receptor ligand as a tracer for the diagnosis of pituitary adenoma ("Small-animal PET with a σ-ligand, 11C-SA4503, detects spontaneous pituitary tumors in aged rats." J. Nucl. Med. 2013 Aug; 54(8):1377-83). Using positron emission tomography (PET), the authors confirmed that the concentration of ¹¹C-SA4503, a piperazine derivative with high affinity for the σ-1 receptor, labelled with carbon-11, was appreciably higher in the pituitary adenoma than in the adjacent healthy parts of the brain and the plasma.

This study confirmed that the σ-1 receptors are widely expressed by tumour cells and that ligands for this receptor may be used for tumour diagnosis via PET.

The pharmacological importance of the σ-1 receptors is also reflected by the numerous patents and patent applications relating to ligands for this receptor and the therapeutic applications thereof. The review by Collina S. et al. ("Sigma receptor modulators: a patent review", Expert Opin. Ther. Patents, 2013, Vol. 23(5):597-613) provides a compendium of patents published between 1986 and 2012 concerning sigma receptor ligands.

Among the cited documents, US 5,180,729 and WO 92/14463 describe a method for treating drug abuse, cocaine and amphetamine abuse, respectively, by administration of (N-phthalimidoalkyl)piperidine derivatives, which act as sigma receptor antagonists and are free of or have mild dopaminergic receptor-blocking activity.

International patent application WO 06/021462 describes pyrazole compounds as particularly selective inhibitors for the sigma receptor and the use thereof for the treatment or prophylaxis of a sigma receptor-mediated disease. The said compounds are useful in the treatment of pain, in particular neuropathic pain and allodynia. Among the compounds described in WO 06/021462, S1RA (also known by the abbreviation E-52862) is a powerful and selective σ-1 receptor antagonist, which is currently in phase II of clinical development for the treatment of neuropathic pain and for the treatment of acute and chronic pain in the case of patients receiving opioid analgesics.

Among the σ-1 receptor agonists, the compound ANAVEX 2-73 successfully completed phase I of clinical development for the treatment of Alzheimer's disease.

In addition, a potent and selective σ-1 receptor agonist of arylalkanolamine structure was described by Rossi D. et al. in "Identification of a potent and selective σ1 receptor agonist potentiating NGF-induced neurite outgrowth in PC12 cells" (Bioorg. Med. Chem., 2011, Vol. 19, pages 6210-6224).

Compounds of arylalkanolamine structure have been described as antimicrobial agents, in particular as bactericides and fungicides, in US 4,820,717.

WO-A-03/106443 discloses certain 1-phenylalkyl piperazines for use in the treatment of disorders or the CNS caused by serotonergic dysfunction.

### SUMMARY OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

Since the sigma-1 receptor is a pharmacological target of recent discovery, there is a great need to find compounds with affinity for this receptor.

The Applicant has found, surprisingly, that arylalkanolamine compounds have affinity for the sigma-1 receptor.

In particular, the Applicant has found, surprisingly, that the arylalkanolamine compounds of formula (I) have antagonist activity on the sigma-1 receptor.

In a first aspect, the present invention thus relates to arylalkanolamine compounds of formula (I): 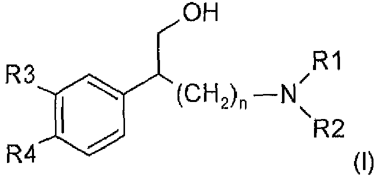 wherein
R1 and R2, equal to or different from each other, are a methyl group, an ethyl group, a benzyl group,or R1 and R2 form, together with the nitrogen atom to which they are bonded, a piperidine or morpholine ring;
R3 and R4, equal to or different from each other, are a hydrogen atom, a phenyl group, a benzyl group, or R3 and R4 form, together with the ring to which they are bonded, an optionally substituted naphthalene ring;
n is an integer from 1 to 5;
and addition salts thereof with pharmaceutically acceptable inorganic and organic acids,
for use as sigma-1 receptor antagonists, in the prevention or treatment of diseases selected from the group consisting of psychotropic substance abuse and pain.

In a second aspect, the present invention relates to a pharmaceutical composition comprising at least one arylalkanolamine compound of formula (I), described above, or an addition salt thereof with pharmaceutically acceptable organic and inorganic acids, and at least one pharmaceutically acceptable excipient, for use in the prevention or treatment of diseases selected from the group consisting of psychotropic substance abuse and pain.

### BRIEF DESCRIPTION OF THE FIGURES

Figures 1, 2 and 3 represent, respectively, the ¹H NMR, ¹³C NMR and IR spectra of compound 1 which is useful according to the present invention, obtained as described in Example 1.
Figures 4 and 5 represent, respectively, the DSC and TGA curves obtained for compound 1 which is useful according to the present invention, obtained as described in Example 1.
Figure 6 represents the results obtained in Example 3.
Figures 7 and 8 represent the results obtained in Example 4.
Figure 9 represents the results obtained in Example 5.

### DETAILED DESCRIPTION OF THE INVENTION

In the present description and in the claims that follow, the term "linear or branched (C₁-C₃)alkyl group" means the methyl, ethyl, n-propyl and isopropyl groups.

In the present description and in the claims that follow, the term "5- or 6-membered heterocyclic ring optionally comprising another heteroatom selected from N, O and S" means, for example, pyrrole, imidazole, pyrazole, pyrroline, pyrrolidine, pyridazoline, imidazoline, imidazolidine, piperidine, piperizine, thiazine and morpholine.

R1 and R2, equal to or different from each other, are a methyl, ethyl or benzyl group, or R1 and R2 form, together with the nitrogen atom to which they are bonded, a piperidine or morpholine ring.

Advantageously, R1 and R2 form, together with the nitrogen atom to which they are bonded, a piperidine ring.

Preferably, R3 and R4, equal to or different from each other, are a hydrogen atom, a phenyl group, or R3 and R4 form, together with the ring to which they are bonded, an optionally substituted naphthalene ring.

Advantageously, the naphthalene ring formed by R3 and R4 is substituted with a hydroxyl group, an alkoxy group having from 1 to 3 carbon atoms, preferably with an alkoxy group having 1 or 2 carbon atoms, for example a methoxy group, or a halogen atom, preferably fluorine.

Advantageously, R3 is hydrogen and R4 is a phenyl group.

Preferably, n is an integer from 1 to 3.

Advantageously, n is 2.

The compounds of formula (I) that are useful according to the present invention are preferably used as addition salts with pharmaceutically acceptable organic or inorganic acids.

Preferably, the pharmaceutically acceptable organic acids are selected from the group consisting of oxalic acid, maleic acid, methanesulfonic acid, para-toluenesulfonic acid, succinic acid, malic acid and tartaric acid.

Preferably, the pharmaceutically acceptable inorganic acids are selected from the group consisting of hydrochloric acid, hydrobromic acid, phosphoric acid and sulfuric acid.

Preferably, the abovementioned compounds of formula (I) are for use in the prevention or treatment of non-opioid psychotropic substance abuse.

Preferably, the said non-opioid psychotropic substances are cocaine and amphetamine.

Preferably, the abovementioned compounds of formula (I) are for use in the treatment of pain, more preferably neuropathic pain and inflammatory pain.

In a non-claimed aspect of the disclosure, the abovementioned compounds of formula (I) are for use in the treatment of cancer, more preferably breast cancer and pituitary adenoma.

In a non-claimed aspect of the disclosure, the abovementioned compounds of formula (I) are for use in the diagnosis of cancer, more preferably breast cancer and pituitary adenoma.

Advantageously, for the diagnosis of cancer, the abovementioned compounds of formula (I) are marked with radioactive isotopes with short half-lives, for example carbon-11 (¹¹C), nitrogen-13 (¹³N), or oxygen-15 (¹⁵O).

Methods for the diagnosis of cancer using compounds labelled with radioactive isotopes are known to those skilled in the art and comprise, for example, positron emission tomography (PET).

Typically, the arylalkanolamine compounds of formula (I) that are useful in the present invention are administered in the form of a pharmaceutical composition.

Preferably, the pharmaceutical composition according to the present invention is prepared in suitable dosage forms comprising an effective amount of at least one compound of formula (I), or of a pharmaceutically acceptable acid-addition salt thereof, and at least one pharmaceutically acceptable excipient.

Examples of suitable dosage forms are tablets, coated tablets, capsules, granules, powders, solutions, suspensions, emulsions, syrups, drops and chewing gums for oral administration; solutions, creams, gels, pastes and ointments for topical administration; medicated patches for transdermal administration; suppositories for rectal administration; and injectable sterile solutions.

Examples of pharmaceutically acceptable excipients are, for example, diluents, binders, glidants, disintegrants, preservatives, stabilizers, buffers, surfactants, solubilizers, salts for regulating the osmotic pressure, emulsifiers, sweeteners, colorants, flavourings and the like.

The amount of the arylalkanolamine compound of formula (I) or of the pharmaceutically acceptable acid-addition salt thereof in the pharmaceutical composition according to the present invention may vary on the basis of factors such as, for example, the type of pathology to be treated, the seriousness of the complaint, the weight of the patient, the dosage form, the selected route of administration, the number of administrations per day and the efficacy of the arylalkanolamine compound of formula (I). However, a person skilled in the art can determine the optimum amount of the compound of formula (I) that is useful according to the present invention in a simple and routine manner.

Typically, the amount of compound of formula (I) or of the pharmaceutically acceptable acid-addition salt thereof to be administered will be such that it ensures a level of administration of from 0.0001 to 100 mg/kg/day. Preferably, the level of administration is from 0.001 to 50 mg/kg/day and more preferably from 0.01 to 10 mg/kg/day.

Preferably, the pharmaceutical composition according to the present invention contains from 0.005 mg to 2 g, more preferably from 0.05 mg to 1.5 g and even more preferably from 0.5 mg to 1 g of at least one compound of formula (I).

Suitable dosage forms for administering the compounds that are useful according to the present invention may be prepared via techniques known in the art and comprise mixing, granulation, compression, dissolution, sterilization and the like.

Examples of the compounds of formula (I) that are useful according to the present invention are represented in Table 2 below.

Compounds 13-16 are for reference only.

**Table 2**

| No. | IUPAC name | Structure |
|---|---|---|
| 1 | 4-(N-piperidinyl)-2-(4-diphenyl)butanol | 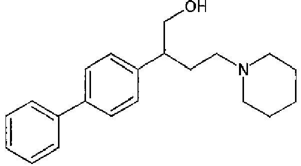 |
| 2 | 4-(N-piperidinyl)-2-[2-naphthyl]butanol | 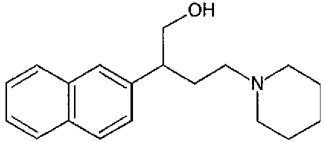 |
| 3 | 4-(N-piperidinyl)-2-[2-(6-methoxy)naphthyl]butanol | 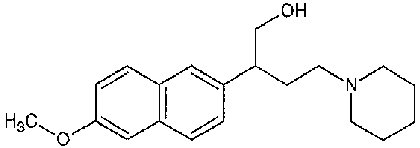 |
| 4 | 4-(N-morpholinyl)-2-(4-diphenyl)butanol | 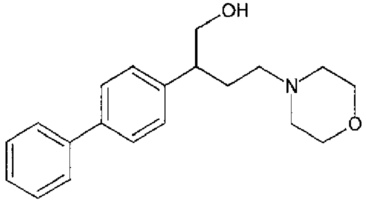 |
| 5 | 4-(N-morpholinyl)-2-[2-naphthyl]butanol | 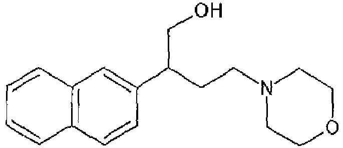 |
| 6 | 4-(N-morphotiny()-2-[2-(6-methoxy)naphthyl]butanol | 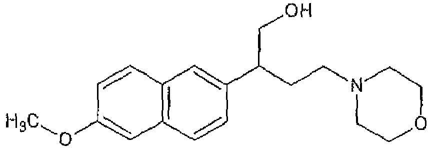 |
| 7 | 4-(N-diethyl)-2-(4-diphenyl)butanol | 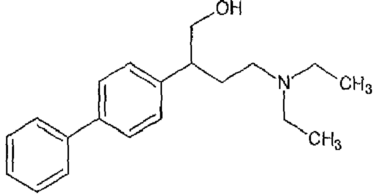 |
| 8 | 4-(N-diethyl)-2-(2-naphthyl)butanol | 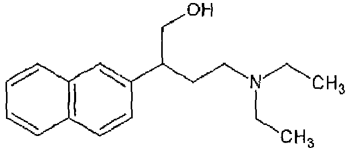 |
| 9 | 4-(N-diethyl)-2-[2-(6-methoxy)naphthyl]butanol | 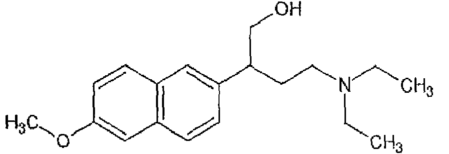 |
| 10 | 4-(N-methyl-N-benzyl)-2-(4-diphenyl)butanol | 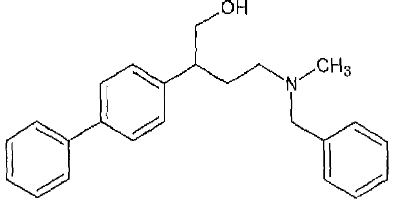 |
| 11 | 4-(N-methyl-N-benzyl)-2-(2-naphthyl)butanol | 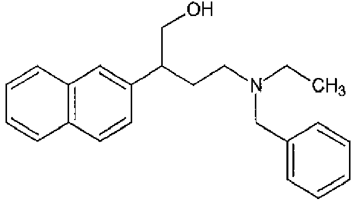 |
| 12 | 4-(N-methyl-N-benzyl)-2-[2-(6-methoxy)naphthyl]butanol | 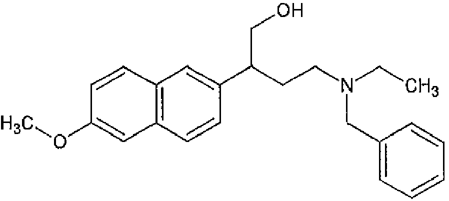 |
| 13 | 4-(N-piperazinyl)-2-(4-diphenyl)butanol | 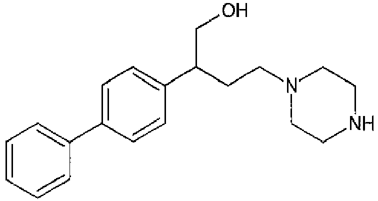 |
| 14 | 4-(N-1,4-thiazinyl)-2-(4-diphenyl)butanol | 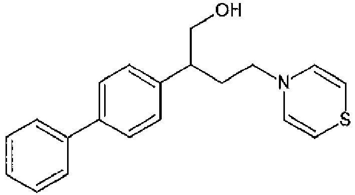 |
| 15 | 4-(N-1,3-imidazolidinyl)-2-(4-diphenyl)butanol | 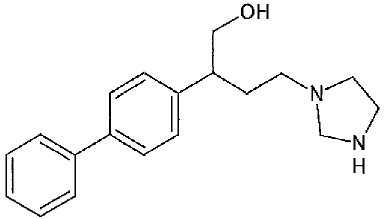 |
| 16 | 4-(N-pyrrolyl)-2-[2-naphthyl]butanol | 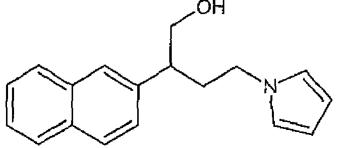 |
| 17 | 4-(N-methyl-N-benzyl)-2-[2-(6-fluoro)-naphthyl]butanol | 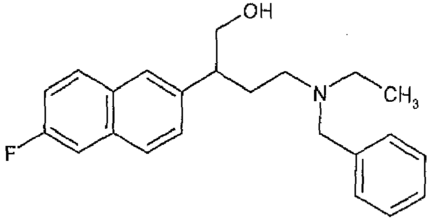 |
| 18 | 4-(N-diethyl)-2-[2-(7-hydroxy)naphthyl]butanol | 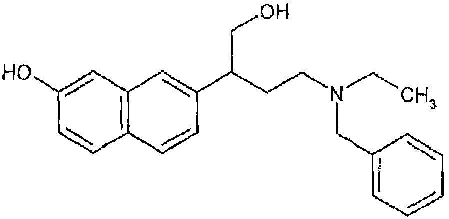 |

The following examples are intended to illustrate the present invention.

### EXAMPLES

### 1. SYNTHESIS OF THE COMPOUNDS OF FORMULA (I)

The compounds of formula (I) may be prepared according to synthetic routes that are known to those skilled in the art, for example as described below.

### General synthesis

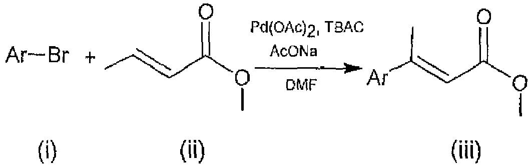

Step (a). Suitable compounds of formula (i) were reacted with methyl crotonate (ii), according to the Heck reaction, using a palladium-based catalyst, for example palladium acetate (Pd(OAc)₂), a strong base, for example sodium acetate (NaOAc), tetrabutylammonium chloride (TBAC) and a suitable reaction solvent, for example N,N-dimethylformamide (DMF). The reaction was performed by heating to a temperature below the boiling point of the solvent, until the intermediate of formula (iii) was obtained.

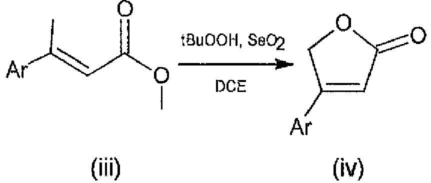

Step (b). The intermediate of formula (iii) was subjected to an allylic oxidation reaction in the presence of tert-butyl peroxide (tBuOOH), using a suitable oxidizing agent, for example selenium dioxide (SeO₂), and a suitable solvent, for example dichloroethane (DCE). The reaction was performed by heating to the boiling point of the solvent, until the intermediate of formula (iv) was obtained.

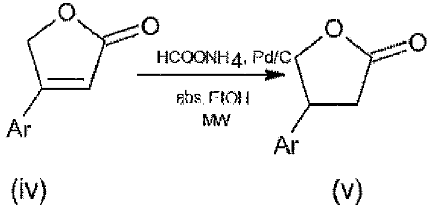

Step (c). The intermediate of formula (iv) was subjected to reduction by hydrogenation, using a suitable metal catalyst, for example 10% palladium-on-charcoal (Pd/C), in the presence of ammonium formate (HCOONH₄) and a suitable solvent, for example absolute ethanol (abs. EtOH), until the intermediate of formula (v) was formed. The reduction reaction may be performed using microwaves (MW) under suitable conditions, for example working between 70-180°C, 100-140 psi and 70-140 W.

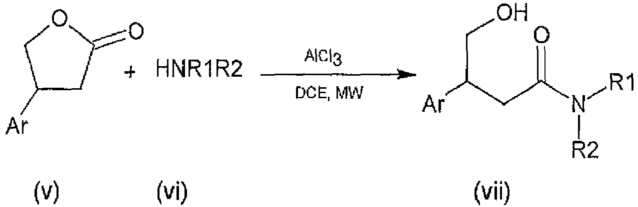

Step (d). The intermediate of formula (v) was reacted with a suitable amine of formula (vi), via an aminolysis reaction, in the presence of aluminium chloride (AlCl₃) and a suitable solvent, for example dichloroethane (DCE), until the amide intermediate of formula (vii) was formed. The aminolysis reaction may be performed using microwaves (MW) under suitable conditions, for example 50-150°C, 100-140 psi and 40-100 W.

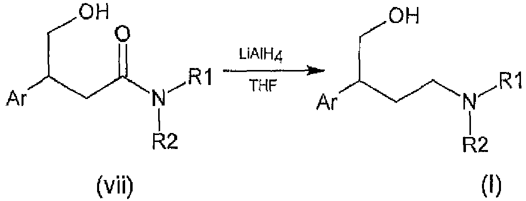

Step (e). The amine intermediate of formula (vii) was subjected to a reduction reaction, using a suitable reducing agent, for example lithium aluminium hydride (LiAlH₄), in a suitable solvent, for example anhydrous tetrahydrofuran (THF). The reaction was performed by working under a nitrogen atmosphere under suitable conditions, for example at 0°C for 6 hours, until the desired final compound of formula (I) was formed.

### Synthesis of the compound of formula 1

Compound 1 was prepared according to the synthetic route described above.

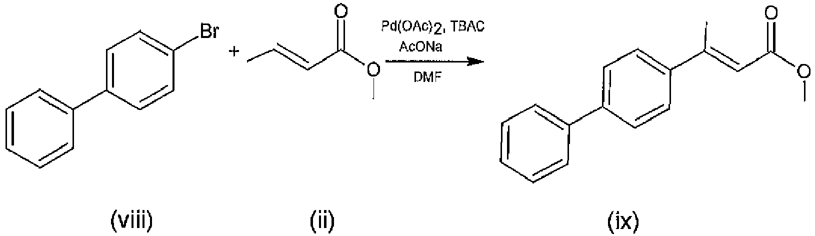

Step (a). The Heck arylation was performed in a two-necked glass round-bottomed flask by reacting 4-bromobiphenyl (viii) (1 equivalent) and an excess of methyl (E)-crotonate (ii) (1.5 equivalents), in the presence of palladium acetate (Pd(OAc)₂ - 0.05 equivalent), tetrabutylammonium chloride (TBAC - 2 equivalents), sodium acetate (NaOAc - 2 equivalents) and using N,N-dimethylformamide (DMF) as solvent. The reaction was performed using an oil bath at a temperature of 120°C for about 5 hours. The reaction product was filtered through Celite, to remove the residual Pd, and the α,β-unsaturated ester of formula (ix) was crystallized from acetone, in a yield of 55%.

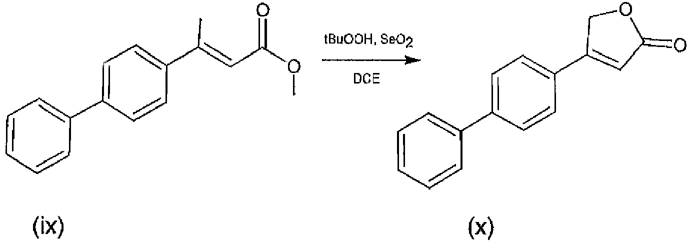

Step (b). The allylic oxidation reaction of the α,β-unsaturated ester of formula (ix) was performed in a two-necked glass round-bottomed flask according to the method proposed by Umbreit and Sharpless (M. A. Umbreit, K. B. Sharpless, "Allylic oxidation of olefins by catalytic and stoichiometric selenium dioxide with tert-butyl hydroperoxide" J. Am. Chem. Soc., 1977, 99 (16), 5526-5528) using tert-butyl hydroperoxide (tBuOOH - 4 equivalents), selenium dioxide (SeO₂ - 0.4 equivalent) as oxidizing agent and dichloroethane (DCE) as reaction solvent, with heating to reflux. The tert-butyl hydroperoxide was added to reoxidize the reduced forms of selenium to SeO₂ and to avoid both the difficulties in removing the colloidal selenium and the formation of reduced forms of selenium. The intermediate of formula (x) was crystallized from 7/3 hexane/ethyl acetate, in a yield of 83%.

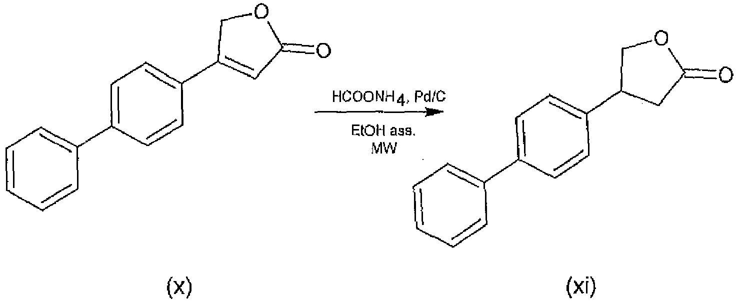

Step (c). The reaction to reduce the double bond in the intermediate of formula (x) was performed in a CEM Discover model microwave oven, using ammonium formate (HCOONH₄ - 2.5 equivalents) as source of molecular hydrogen and 10% Pd/C as catalyst, in absolute ethanol (abs. EtOH). The reaction was performed using microwaves (MW) with a power of 100 watts, at 100°C and 120 psi. These conditions made it possible to obtain the intermediate of formula (xi) in a reaction time of 90 seconds and in a yield of 100%.

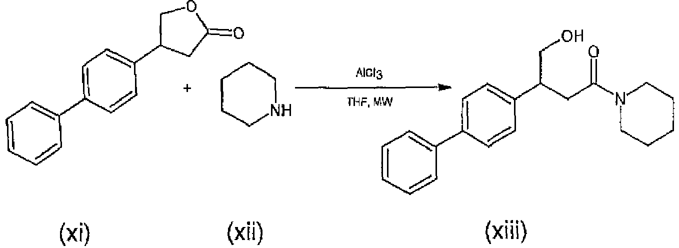

Step (d). The aminolysis reaction was performed in a CEM Discover model microwave oven, by heating with microwaves (MW) with a power of 120 watts, at 100°C and 120 psi for 25 minutes, using 1 equivalent of the intermediate of formula (xi), piperidine (xii - 2.5 equivalents), AlCl₃ (1.3 equivalents) and THF as reaction solvent. The compound of formula (xiii) was purified by automated flash chromatography (Isolera Biotage), in a yield of 61%.

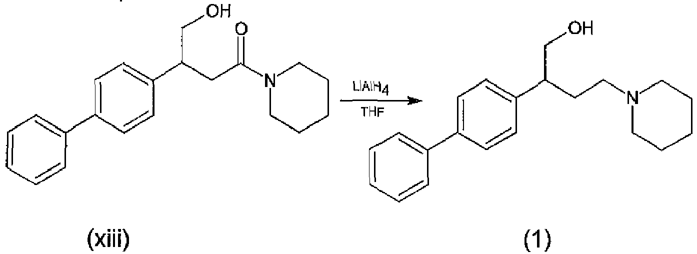

Step (e). The intermediate of formula (xiii) was subjected to reduction, by adding LiAlH₄ (3 equivalents) in anhydrous THF (distilled over Na° and benzophenone). The reaction was performed in a two-necked glass round-bottomed flask under an inert atmosphere of nitrogen and at room temperature for 20 hours. At the end of the reaction, compound (1) was obtained as a white solid, in a yield of 66%.
Melting point: 221.6-222.3°C

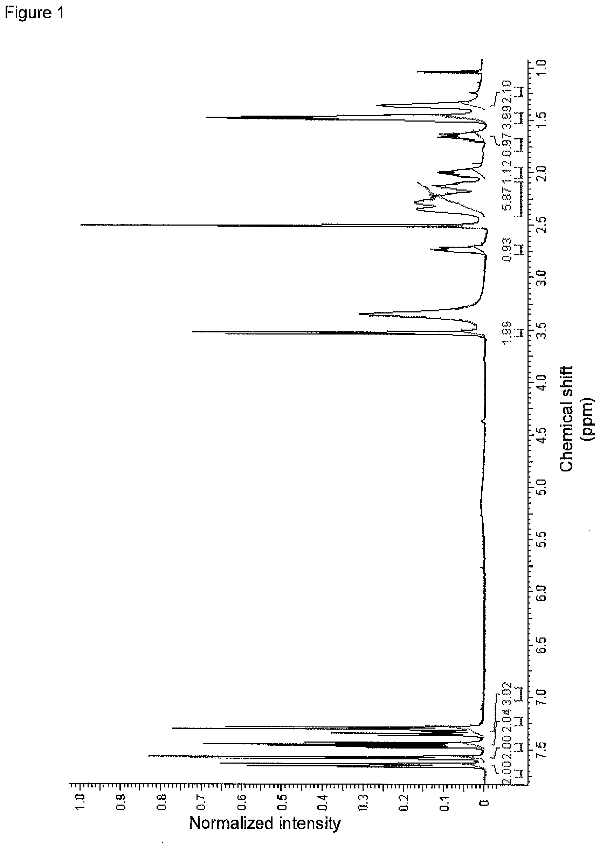

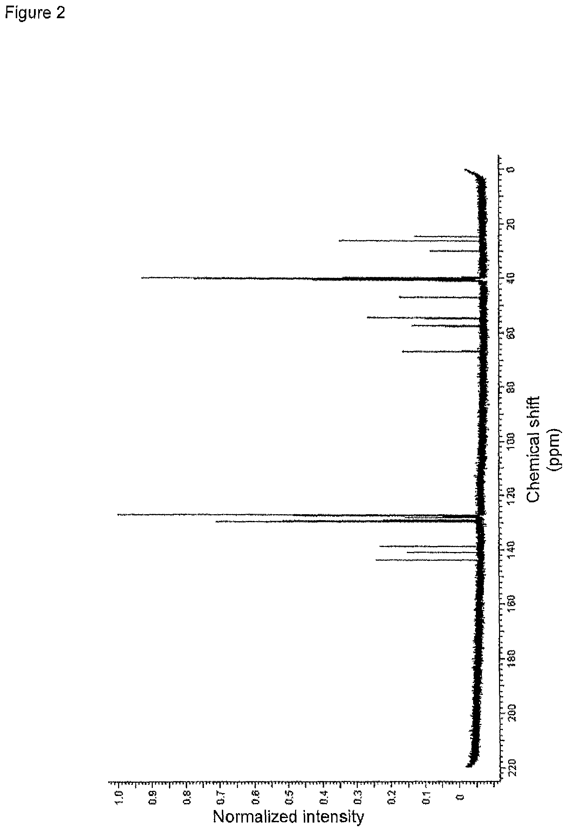

The nuclear magnetic resonance spectra (¹H NMR and ¹³C NMR) obtained for compound 1 are reported, respectively, in Figures 1 and 2. In the spectra, the chemical shift (in ppm) is reported on the x-axis and the normalized intensity is reported on the y-axis.

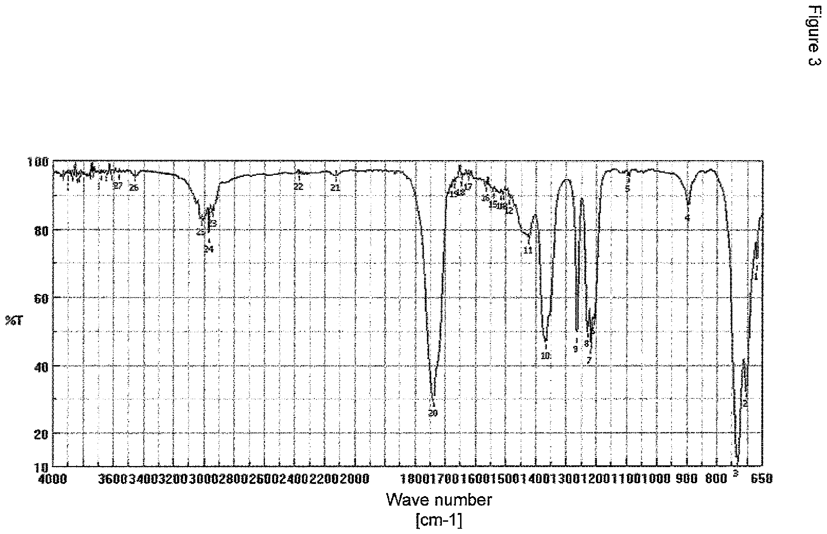

The infrared (IR) spectrum of compound 1 is reported in Figure 3, in which the wavelength (expressed in cm⁻¹) is reported on the x-axis and the percentage of transmittance (%T) is reported on the y-axis.

The solid state of the compound of formula 1 was characterized using thermal analytical techniques, in particular differential scanning calorimetry (DSC) and simultaneous thermogravimetric analysis (TGA/DSC), as described below.

Differential scanning calorimetry (DSC). The temperature and enthalpy values were measured using the Mettler STARe system (Mettler Toledo, Novate Milanese, Mi, IT), equipped with DSC821e Module and Intracooler devices for analysis at sub-ambient temperature (Julabo FT 900) on samples of 2-4 mg (Mettler M3 Microbalance) in aluminium containers sealed with perforated lids.

Simultaneous thermogravimetric analysis (TGA/DSC). The losses of mass were recorded with a Mettler STARe system (Mettler Toledo, Novate Milanese, Mi, IT) by TGA with simultaneous DSC analysis on samples of 4-6 mg placed in open aluminium crucibles.

Both analyses were performed under a nitrogen atmosphere (flow rate of 50 mL/minute), using a temperature variation of 10°C/minute, over the temperature range from 30°C to 300°C. The instruments were precalibrated using indium as reference product. The analyses were performed in triplicate.

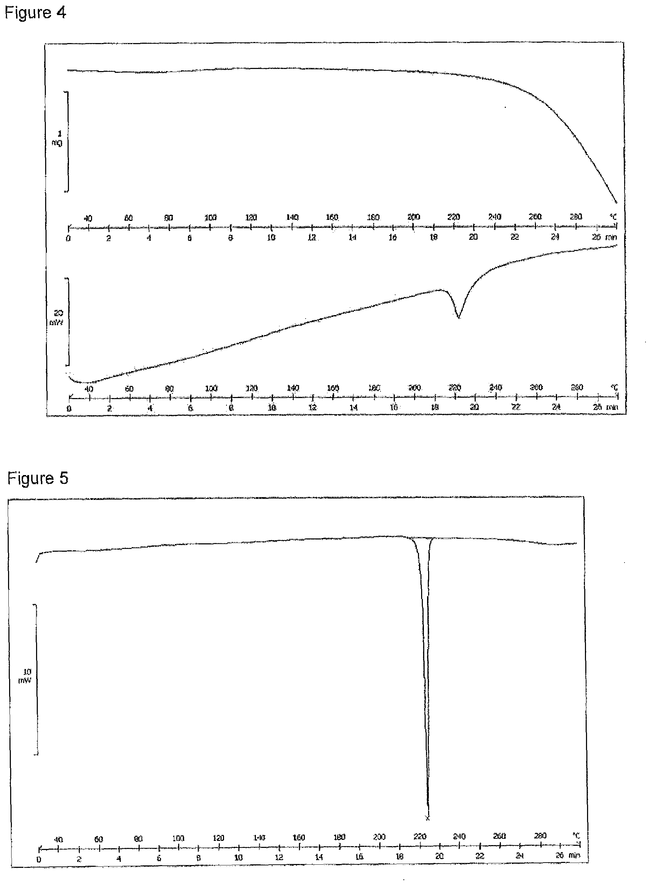

The spectra obtained by DSC analysis and TGA/DSC analysis are reported, respectively, in Figures 4 and 5.

### Synthesis of the compounds of formulae 2-18

Compounds 2-18 were prepared using the synthetic route described for compound 1, using the intermediates reported in Table 3 below:

| Table 3 | | |
|---|---|---|
| No. | Step (a), intermediate (i) | Step (d), amine (vi) |
| 2 | 2-bromonaphthyl | piperidine |
| 3 | 2-bromo(6-methoxy)naphthyl | piperidine |
| 4 | 4-bromobiphenyl | morpholine |
| 5 | 2-bromonaphthyl | morpholine |
| 6 | 2-bromo(6-methoxy)naphthyl | morpholine |
| 7 | 4-bromobiphenyl | N,N-diethylamine |
| 8 | 2-bromonaphthyl | N,N-diethylamine |
| 9 | 2-bromo(6-methoxy)naphthyl | N,N-diethylamine |
| 10 | 4-bromobiphenyl | N-methyl-N-benzylamine |
| 11 | 2-bromonaphthyl | N-methyl-N-benzylamine |
| 12 | 2-bromo(6-methoxy)naphthyl | N-methyl-N-benzylamine |
| 13 | 4-bromobiphenyl | piperazine |
| 14 | 4-bromobiphenyl | 1,4-thiazine |
| 15 | 4-bromobiphenyl | imidazolidine |
| 16 | 2-bromonaphthyl | pyrrole |
| 17 | 2-bromo(6-fluoro)naphthyl | N-methyl-N-benzylamine |
| 18 | 2-bromo(7-hydroxy)naphthyl | N,N-diethylamine |

### 2. TEST OF BINDING TO THE SIGMA-1 RECEPTOR

The general protocol for the binding test, the guinea pig brain membrane homogenate and the method for the quantitative determination of the receptors were arranged as described by Rossi D. et al. in "Chemical, pharmacological, and in vitro metabolic stability studies on enantiomerically pure RC-33 compounds: promising neuroprotective agents acting as σ1 receptor agonist" (ChemMedChem 2013, 8, 1514-1527).

The affinity of compound 1 for the σ-1 receptor was determined by competition with the radioligand [³H](+)pentazocin (22.0 Ci/mmol; Perkin-Elmer), which has high selectivity for the σ-1 receptor.

The guinea pig cerebral cortex membrane homogenate (containing about 100 mg of σ-1 receptor) was thawed and incubated with compound 1 at various concentrations, with [³H](+)pentazocin (2 nM) and Tris buffer (50 mm, pH 7.4) at 37°C.

The non-specific binding was determined in the presence of an excess of unlabelled (+)-pentazocin (10 mM, Kd = 2.9 nM). The IC₅₀ values obtained from three independent experiments were transformed into the respective affinity constants (Kᵢ), using the Cheng-Prusoff equation.

Compound 1 showed a Kᵢ value ± 6.2 nM.

In general, compounds with a Kᵢ value of less than or equal to 25 nM were considered as having affinity for the sigma-1 receptor. Compounds with a Kₗ value of less than or equal to 10 nM were considered as having particularly good affinity for the sigma-1 receptor and, therefore, as highly promising.

### 3. ANTAGONIST ACTIVITY ON THE SIGMA-1 RECEPTOR

Evaluation of the growth of neurite outgrowths induced by neuronal growth factor (NGF).

Evaluation of the growth of NGF-induced neurite outgrowths was performed on rat pheochromocytoma cells (PC12 cells), conventionally used as a model of neuronal study.

This method constitutes a reliable model for evaluating the agonist/antagonist profile of low molecular weight compounds on the σ receptors. In particular, it has been reported that σ-1 agonists potentiate the growth of NGF-induced neurite outgrowths in PC12 cells. In contrast, σ-1 antagonists oppose the action of the agonists, leading to a reduction in NGF-induced growth (Rossi D. et al., "Identification of a potent and selective σ1 receptor agonist potentiating NGF-induced neurite outgrowth in PC12 cells", Bioorg. Med. Chem. 2011, 19, 6210-6224).

The cell culturing PC12 cells, the method for quantifying the growth of neurite outgrowths and the cytotoxicity test were performed as described by Rossi D. et al. in Bioorg. Med. Chem. 2011 cited above.

Statistical analysis of the data obtained was performed using the two-way analysis of variants (ANOVA) method and the Tukey post-hoc test. Values of p < 0.05 were considered as being statistically significant.

The results, expressed as the mean value ± standard error of mean (SEM), represent the percentage of cells showing neurite growth.

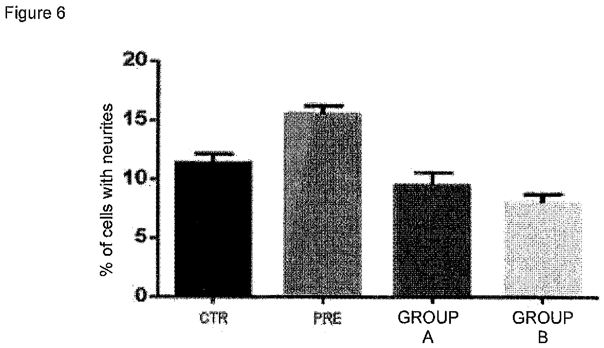

Figure 6 represents the results obtained for the following groups:
- control group (CTR): cells treated with NGF;
- reference group (PRE): cells treated with 2-(4-morpholinoethyl)-1-phenylcyclohexanecarboxylate hydrochloride with σ-1 agonist activity;
- group A: cells treated with 2-(4-morpholinoethyl)-1-phenylcyclohexanecarboxylate hydrochloride in the presence of 2.5 µM of compound 1; and
- group B: cells treated with 2-(4-morpholinoethyl)-1-phenylcyclohexanecarboxylate hydrochloride in the presence of 10 µM of compound 1.

The data presented in Figure 6 clearly show that the percentage of cells with growth of neurite outgrowths increased significantly in the reference group (PRE), treated with the reference compound with agonist activity on the σ-1 receptors, relative to the control group.

Simultaneous treatment of PC12 cells with compound 1, at a concentration even of 2.5 µM (group A), appreciably opposed the effect of the agonist compound, to the extent that the number of cells with growth of neurite outgrowths decreased even relative to the control group (CTR). The effect was even more pronounced using compound 1 at a concentration of 10 µM (group B).

This test made it possible to demonstrate the antagonist activity on σ-1 receptors of the compounds of formula (I) that are useful according to the present invention.

### 4. IN VITRO METABOLIC STABILITY

The metabolic stability studies were performed using biological matrices (plasma, blood and S9 fraction of liver) obtained from various animal species.

The biological matrices and the analysis method were arranged according to the process described by Rossi D. et al. in "Identification of RC-33 as a potent and selective σ1 receptor agonist potentiating NGF-induced neurite outgrowth in PC12 cells. Part 2: g-scale synthesis, physicochemical characterization and in vitro metabolic stability" (Bioorg. Med. Chem. 2013, 21, 2577-2586).

For each biological matrix, suitable solutions were prepared to perform the test, as described below.

10 µL of a stock solution of compound 1 in dimethyl sulfoxide (DMSO) were incubated with each matrix (total reaction volume 1 mL) in a mixer thermostatically maintained at 37°C for 2 hours. 50 µL aliquots were taken from each reaction mixture and sampled in duplicate at 5, 10, 15, 30, 60, 120 and 240 minutes to determine the reaction kinetics. The reaction was stopped by adding acetonitrile (150 µL) and DMSO (10 µL) to precipitate the protein. The samples were mixed using a vortex mixer and centrifuged at 4000 rpm for 10 minutes at 4°C. The supernatant was analysed by means of an Acquity Waters ultra-fast liquid chromatography system, interfaced with a photodiode array detector (UPLC/UV/PAD) to obtain the concentration of the test compound at each of the said times. The extrapolated concentration was used to calculate the half life (t_{1/2}) of compound 1 in each matrix.

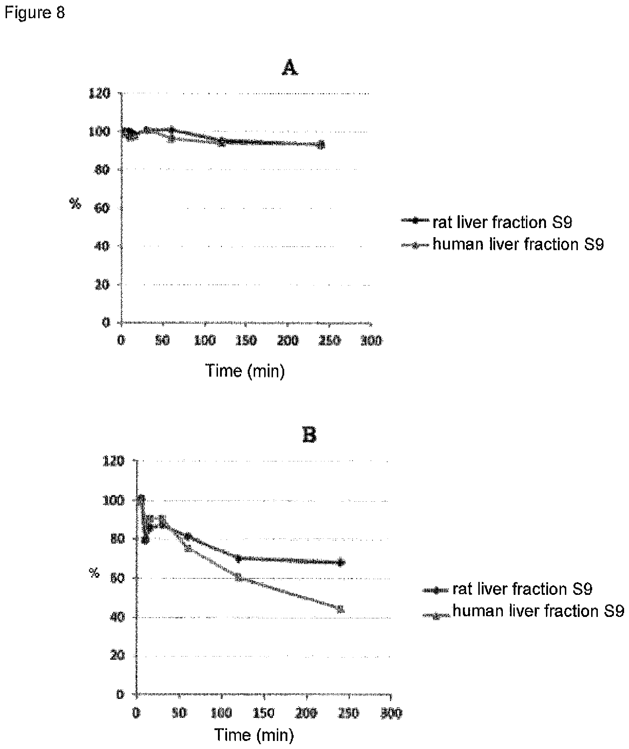

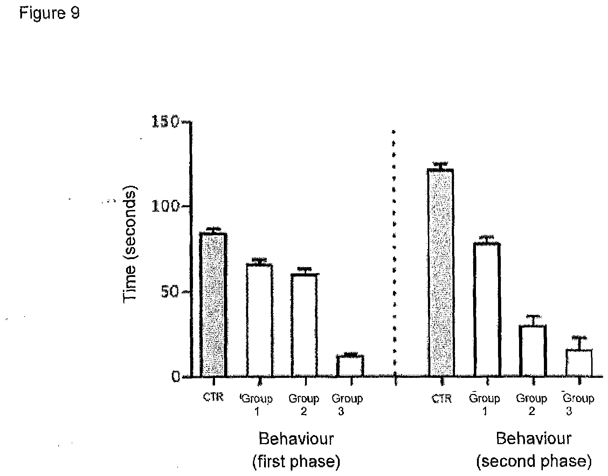

The results, obtained from three independent experiments and expressed as the mean value ± standard deviation (SD), are given in Figures 8 and 9.

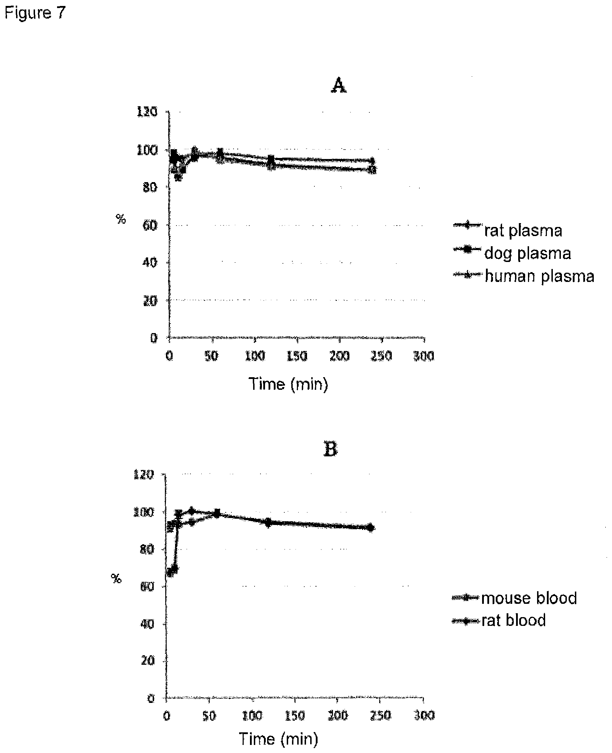

In particular, Figure 7(A) shows that the metabolization of compound 1 in rat, dog and human plasma was insignificant. Similarly, Figure 7(B) shows that the metabolization of compound 1 in mouse and rat blood was insignificant.

Figure 8(A) shows that the metabolization of compound 1 in S9 fraction of rat and human liver in the absence of NADPH was insignificant.

Finally, Figure 8(B) shows that compound 1 undergoes substantial metabolization in S9 fraction of rat and human liver in the presence of NADPH.

The results obtained made it possible to conclude that compound 1 has high *in vitro* metabolic stability. On the basis of these results, it was also possible to hypothesize that compound 1 that is useful according to the present invention has high *in vivo* stability.

### 5. FORMALIN TEST

The formalin test is an animal model of inflammatory pain widely used and known in the literature.

32 male ICR mice weighing 25 grams were divided into 4 groups of 8 mice each.

The vehicle alone, which consisted of a mixture of dimethyl sulfoxide (DMSO), castor oil Cremophor® EL (BASF) and distilled water, in a 10/10/80 ratio, was administered to the control group (CTR).

Compound 1 was dissolved in the same vehicle and administered at the following doses:
- group 1, 1 mg/kg;
- group 2, 10 mg/kg; and
- group 3, 20 mg/kg.

All the administrations took place by intraperitoneal injection. Thirty minutes after the administration, all the mice were subjected to an injection with 20 uL of a 2% formalin solution subcutaneously, into a paw.

The injection of formalin induces a two-phase nociceptive response, in other words, within the first 5-10 minutes of the administration of formalin, the first nociceptive phase is manifested, followed by a phase of quiescence and by the second nociceptive phase which is manifested 20-30 minutes after the administration of formalin. In addition, the formalin-induced nociceptive response is spontaneous, i.e. after the injection the animal shows typical behaviour due to pain (for example, it licks and bites the paw), without it being necessary to solicit the injection zone with a nociceptive stimulus (McNamara C.R. et al, "TRPA1 mediates formalin-induced pain." PNAS, 14 August 2007, Vol. 104(33):13525-13530).

The nociceptive response was recorded by evaluating the time that each mouse spent licking or biting its paw which received the injection. The evaluation took place in the interval from 0 to 5 minutes after the administration of formalin (first phase) and in the interval from 20 to 30 minutes after the administration of formalin (second phase).

Figure 9 shows the results obtained, expressed as the mean time (in seconds) that each group of mice spent licking or biting the paw which received the injection of formalin in the first and in the second phases.

The results obtained made it possible to conclude that compound 1 is effective in reducing inflammatory pain even at a dose of 1 mg/kg.

## Claims

1. Aryl alkanolamine compounds having genera! formula (I): 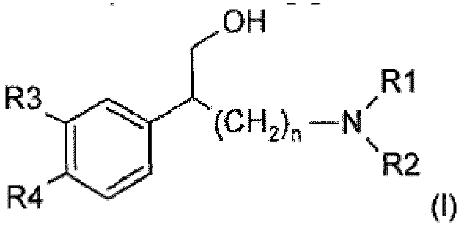 wherein
R1 and R2, equal to or different from each other, are a methyl group, an ethyl group, a benzyl group,
or R1 and R2 form, together with the nitrogen atom to which they are bonded, a piperidine or morpholine ring
R3 and R4, equal to or different from each other, are hydrogen atom, a phenyl group, a benzyl group
or R3 and R4 form, together with the ring to which they are bonded, an optionally substituted naphthalene ring; and
n is an integer from 1 to 5;
and addition salts thereof with pharmaceutically acceptable inorganic and organic acids,
for use, as sigma-1 receptor antagonists, in the prevention or treatment of diseases selected from the group consisting of psychotropic substance abuse and pain.

2. The aryl alkanolamine compounds for use according to claim 1, wherein R1 and R2 form, together with the nitrogen atom to which they are bonded, a piperidine ring.

3. The aryl alkanolamine compounds for use according to claim 1, for the use in the prevention or treatment of non-opioid psychotropic substance abuse.

4. The aryl alkanolamine compounds for use according to claim 3, wherein said non-opioid psychotropic substances are cocaine and amphetamines.

5. The aryl alkanolamine compounds for use according to claim 1, for the use in the prevention or treatment of neuropathic pain or inflammatory pain.

6. A pharmaceutical composition comprising an aryl alkanolamine compound according to claim 1 or 2
or an addition salt thereof with pharmaceutically acceptable inorganic and organic acids,
and at least one pharmaceutically acceptable excipient,
for use in the prevention or treatment of diseases selected from the group consisting of psychotropic substance abuse and pain.

7. The pharmaceutical composition for use according to claim 6, wherein said at least one pharmaceutically acceptable excipient is selected from the group consisting of diluents, binders, glidants, disintegrants, preservatives, stabilizers, buffers, surfactants, solubilizers, salts for regulating the osmotic pressure, emulsifiers, sweeteners, colorants, and flavourings.

8. The pharmaceutical composition for use according to claims 6 or 7, wherein said composition comprises an amount of from 0.005 mg to 2 g of said aryl alkanolamine compound of formula (I).

9. The pharmaceutical composition for use according to claim 8, wherein said composition comprises an amount of from 0.05 mg to 1 .5 g of said aryl alkanolamine compound of formula (I).

10. The pharmaceutical composition for use according to claim 9, wherein said composition comprises an amount of from 0.5 mg to 1 g of said aryl alkanolamine compound of formula (I).

11. The pharmaceutical composition for use according to claim 6, for the use in the prevention or treatment of non-opioid psychotropic substance abuse.

12. The pharmaceutical composition for use according to claim 11, wherein said non-opioid psychotropic substances are cocaine and amphetamine.

13. The pharmaceutical composition for use according to claim 6, for the use in the prevention or treatment of neuropathic pain or inflammatory pain.

## Patentansprüche

1. Arylalkanolamin-Verbindungen mit der allgemeinen Formel (I): 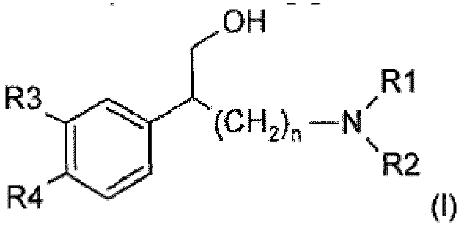 wobei
R1 und R2, gleich zueinander oder verschieden voneinander, eine Methylgruppe, eine Ethylgruppe, eine Benzylgruppe sind,
oder R1 und R2 zusammen mit dem Stickstoffatom, an das sie gebunden sind, einen Piperidin- oder Morpholinring bilden
R3 und R4, gleich zueinander oder verschieden voneinander, ein Wasserstoffatom, eine Phenylgruppe, eine Benzylgruppe sind
oder R3 und R4 zusammen mit dem Ring, an den sie gebunden sind, einen gegebenenfalls substituierten Naphthalinring bilden; und
n eine ganze Zahl von 1 bis 5 ist;
und Additionssalze davon mit pharmazeutisch annehmbaren anorganischen und organischen Säuren,
zur Verwendung als Sigma-1-Rezeptor-Antagonisten bei der Prävention oder Behandlung von Krankheiten ausgewählt aus der Gruppe bestehend aus Abusus von psychotropen Substanzen und Schmerz.

2. Arylalkanolamin-Verbindungen zur Verwendung nach Anspruch 1, wobei R1 und R2 zusammen mit dem Stickstoffatom, an das sie gebunden sind, einen Piperidinring bilden.

3. Arylalkanolamin-Verbindungen zur Verwendung nach Anspruch 1 zur Verwendung bei der Prävention oder Behandlung von Abusus von nicht-opioiden psychotropen Substanzen.

4. Arylalkanolamin-Verbindungen zur Verwendung nach Anspruch 3, wobei die besagten nicht-opioiden psychotropen Substanzen Kokain und Amphetamine sind.

5. Arylalkanolamin-Verbindungen zur Verwendung nach Anspruch 1 zur Verwendung bei der Prävention oder Behandlung von neuropathischen Schmerzen oder entzündlichen Schmerzen.

6. Pharmazeutische Zusammensetzung, umfassend eine Arylalkanolamin-Verbindung nach Anspruch 1 oder 2
oder ein Additionssalz davon mit pharmazeutisch annehmbaren anorganischen und organischen Säuren,
und mindestens einen pharmazeutisch annehmbaren Hilfsstoff,
zur Verwendung bei der Prävention oder Behandlung von Krankheiten ausgewählt aus der Gruppe bestehend aus Abusus von psychotropen Substanzen und Schmerz.

7. Pharmazeutische Zusammensetzung zur Verwendung nach Anspruch 6, wobei der besagte mindestens einen pharmazeutisch annehmbaren Hilfsstoff ausgewählt ist aus der Gruppe bestehend aus Verdünnungsmitteln, Bindemitteln, Gleitmitteln, Abbaumitteln, Konservierungsmitteln, Stabilisatoren, Puffern, Tensiden, Lösungsvermittlern, Salzen zur Regulierung des osmotischen Drucks, Emulgatoren, Süssstoffe, Farbstoffe und Aromen.

8. Pharmazeutische Zusammensetzung zur Verwendung nach den Ansprüchen 6 oder 7, wobei die besagte Zusammensetzung eine Menge von 0.005 mg bis 2 g der besagten Arylalkanolamin-Verbindung der Formel (I) umfasst.

9. Pharmazeutische Zusammensetzung zur Verwendung nach Anspruch 8, wobei die besagte Zusammensetzung eine Menge von 0.05 mg bis 1 .5 g der besagten Arylalkanolamin-Verbindung der Formel (I) umfasst.

10. Pharmazeutische Zusammensetzung zur Verwendung nach Anspruch 9, wobei die besagte Zusammensetzung eine Menge von 0.5 mg bis 1 g der besagten Arylalkanolamin-Verbindung der Formel (I) umfasst.

11. Pharmazeutische Zusammensetzung zur Verwendung nach Anspruch 6, zur Verwendung bei der Prävention oder Behandlung von Abusus von nicht-opioiden psychotropen Substanzen.

12. Pharmazeutische Zusammensetzung zur Verwendung nach Anspruch 11, wobei die besagten nicht-opioiden psychotropen Substanzen Kokain und Amphetamine sind.

13. Pharmazeutische Zusammensetzung zur Verwendung nach Anspruch 6, zur Verwendung bei der Prävention oder Behandlung von neuropathischen Schmerzen oder entzündlichen Schmerzen.

## Revendications

1. Composés d'aryl alkanolamine de formule générale (1) : 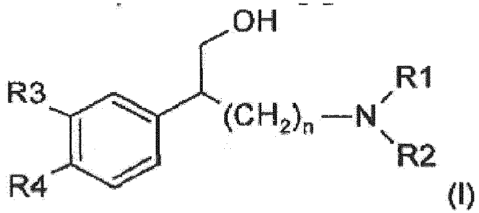 dans laquelle
R1 et R2, similaires ou différents l'un de l'autre représentent un groupe méthyl, un groupe éthyl, un groupe benzyl,
ou R1 et R2 forment, conjointement avec l'atome d'azote auquel ils sont liés un cycle pipéridine ou morpholine,
R3 et R4, similaires ou différents l'un de l'autre représentent un atome d'hydrogène, un groupe phényl, un groupe benzyl,
ou R3 et R4 forment, conjointement avec le cycle auquel ils sont liés un cycle naphtalène, le cas échéant substitué, et
n représente un nombre entier de 1 à 5, et
leurs sels d'addition avec des acides inorganiques et organiques pharmaceutiquement acceptables,
destinés à être utilisés en tant qu'antagonistes du récepteur sigma 1 pour la prévention ou le traitement de maladies choisies dans le groupe formé par l'abus de substances psychotropes et la douleur.

2. Composés d'aryl alkanolamine destiné à être utilisé conformément à la revendication 1, dans lesquels R1 et R2 forment, conjointement avec l'atome d'azote auquel ils sont liés, un cycle pipéridine.

3. Composés d'aryl alkanolamine destinés à être utilisé conformément à la revendication 1, destinés à être utilisé pour la prévention ou le traitement de l'abus de substances psychotropes non opiacées.

4. Composés d'aryl alkanolamine destinés à être utilisé conformément à la revendication 3, dans lesquels les substances psychotropes non opiacées sont la cocaïne et des amphétamines.

5. Composés d'aryl alkanolamine destinés à être utilisés conformément à la revendication 1, destinés à être utilisés pour la prévention ou le traitement de douleurs neuropathiques ou de douleurs inflammatoires.

6. Composition pharmaceutique renfermant un composé d'aryl alkanolamine conforme à la revendication 1 ou 2,
ou un sel d'addition de celui-ci avec des acides organiques et inorganiques pharmaceutiquement acceptables, et
au moins un excipient pharmaceutiquement acceptable,
destiné à être utilisé pour la prévention ou le traitement de maladies choisies dans le groupe formé par l'abus de substances psychotropes et la douleur.

7. Composition pharmaceutique destinée à être utilisée conformément à la revendication 6, dans laquelle l'excipient pharmaceutiquement acceptable est choisi dans le groupe formé par des diluants, des liants, des agents de glissement, des agents de désintégration, des agents de préservation, des agents stabilisants, des tampons, des agents tensioactifs, des agents de solubilisation, des sels permettant de réguler la pression osmotique, des agents émulsifiants, des agents adoucissants, des colorants et des agents d'aromatisation.

8. Composition pharmaceutique destinée à être utilisée conformément à la revendication 6 ou 7, renfermant une quantité de 0,005 mg à 2 g du composé d'aryl alkanolamine conforme à la formule (1).

9. Composition pharmaceutique destinée à être utilisée conformément à la revendication 8, renfermant une quantité de 0,05 mg à 1,5 g du composé d'aryl alkanolamine conforme à la formule (1).

10. Composition pharmaceutique destinée à être utilisée conformément à la revendication 9, renfermant une quantité de 0,5 mg à 1 g du composé d'aryl alkanolamine conforme à la formule (1).

11. Composition pharmaceutique destinée à être utilisée conformément à la revendication 6, destinée à être utilisée pour la prévention ou le traitement de l'abus de substances psychotropes non opiacées.

12. Composition pharmaceutique destinée à être utilisée conformément à la revendication 11, dans laquelle les substances psychotropes non opiacées sont la cocaïne et des amphétamines.

13. Composition pharmaceutique destinée à être utilisée conformément à la revendication 6, destinée à être utilisée pour la prévention ou le traitement de douleurs neuropathiques ou de douleurs inflammatoires.
